# EUROPEAN PATENT APPLICATION

(11) **EP 2 479 691 A1**
(43) Date of publication of application: **25.07.2012**
(21) Application number: 11151683.7
(22) Date of filing: 21.01.2011
(51) Int. Cl.: G06F 19/00

(54) **Pharmaceutical product and communication tool**

(71) Applicant: Cederlund, Johan, 16855 Bromma (SE)
(72) Inventor: Cederlund, Johan, 16855 Bromma (SE)
(74) Representative: Rystedt, Per Hampus

(57) **Abstract**

The invention relates to a kit of parts comprising
- a pharmaceutical product; and
- a computer program product comprising instructions causing a computer to perform a method comprising the steps
o providing a patient with a set of questions according to a question schedule, wherein said set of questions is specific to the pharmaceutical product;
o collecting answers to said questions from said patient;
o subjecting said answers to a set of functions specific for the set of questions and the pharmaceutical product thereby generating patient-specific feedback information; and

providing said feedback information to the patient.

The invention furthermore relates to the computer program product as such and to the method performed by the computer program product.

## Description

### Field of the invention

The present invention relates to the field of drug administration, and particularly to combination products for management and follow-up of drug administration.

### Background

Drugs on the market today are thoroughly tested with regard to their efficacy and safety during extensive clinical trials before they are approved for marketing by a national or regional Medical Products Agency, such as EMA in Europe or FDA in the U.S.

An important aspect of the clinical trials is to achieve an optimal dosage and administration regimen and these aspects are strictly controlled and monitored during the trials. During clinical trials the manufacturers of a drug collect a large amount of data on the drug. However, once the drug is on the market the control of the dosage is in many cases left to the patient undergoing therapy. This may lead to lack of compliance to the prescribed dosing, such as under-dosage, over-dosage and gaps in the administration, which leads to less than satisfactory results of the treatment.

Medical devices enhancing the therapeutic effect of drugs are known. For instance, specifically designed inhalers are used to administer various anti-asthmatic drugs and implantable devices have been used for controlled release of anti-cancer drugs.

Patient compliance and monitoring systems are known in the art, e.g. WO02095352. Such systems are focused on monitoring patient compliance and reporting to the medical practitioner and the patient how the treatment is progressing. The system disclosed in WO02095352 is relevant for a certain condition (menopause) and a general therapy (hormone replacement therapy). It is not specifically adapted for a certain pharmaceutical product.

Patient-reported outcome (PRO) measures are used in medical product development and sometimes used to support labelling claims, see U.S. Department of Health and Human Services Food and Drug Administration. Guidance for Industry. Patient-Reported Outcome Measures: use in Medical Product Development to Support Labelling Claims, 2009, http://www.fda.gov/downloads/Drugs/GuidanceComplianceRegulatoryInformation/Guidance s/UCM193282.pdf.

### Summary of the invention

The present invention is based on the realization that the integral combination of a pharmaceutical product and a specifically adapted system for receiving information from a user of the pharmaceutical product and providing feedback to said user can be used to achieve a number of benefits in clinical practice.

The main aspect of the invention is a combination product, or a kit-of-parts, comprising the drug in question and a computer program product comprising instructions causing a computer to provide the patient with the questions, receiving answers to the questions, processing the answers and providing feedback to the patient.

One aspect of the invention is to enhance patient compliance to the prescribed dosage or administration regimen and to enhance the clinical efficacy of the pharmaceutical product. This may be done by including questions on the actual administration; actual dosage; perceived and/or measured therapeutic effects; test results and/or perceived quality of life and providing the patient with feedback correlating the positive effects of the pharmaceutical product, and/or the absence or low prevalence of negative effects, with compliance to the prescribed dosage or administration regimen.

One aspect of the invention is to give the user early indications of the occurrence or development of a possible adverse event and/or side effect, by including questions relating to occurrence or development of a possible adverse event and/or side effect. This increased awareness of adverse events and side effects results in enhanced protection of patients from adverse events and side effects. This may enable early introduction of pharmaceutical products with an incomplete safety profile on the market, since it allows for making each user of the pharmaceutical product aware of the occurrence or development of a possible adverse event and/or side effect and also facilitates that this may be reported directly to medical staff. It may also enable re-introduction of products withdrawn from the market due to an unacceptably high frequency of adverse events or side effects by making each user of the pharmaceutical product aware of the occurrence or development of a possible adverse event and/or side effect at an early stage.

One aspect of the invention is to enhance the patient's quality of life.

The computer program product is preferably adapted to be installed on a handheld device, such as a mobile telephone, a Personal Digital Assistant (PDA) or similar devices. The computer program product may also be installed on a remote computer, e.g. a. server, web or cloud-based service, and accessible to the user through a computer such as a handheld device, a stationary computer, a laptop or the like. In such a case the feedback is also preferably provided through the same computer.

Other aspects of the invention is the computer program product itself and the method performed by the computer program product.

### Brief description of the drawings

Figure 1 is a schematic overview of the combination product according to the invention.
Figure 2 shows an alternative embodiment of the invention.
Figure 3 shows an alternative embodiment of the invention.

### Definitions

All words and terms used in the present specification are intended to have the meaning usually given to them in the relevant art. However, for the sake of clarity, a few terms are specifically defined below.

The term "set of questions" is a questionnaire with predetermined questions or items shown to a respondent to get answers for feedback purposes. The questions within the set preferably have a limited number of possible answers, such as yes/no; scale 1-10; multiple choice; etc. The questions may however also have an undefined number of answers, such as a value of a test parameter (e.g. blood pressure, blood glucose level).

The questions in the set of question is posed to the respondent according to a certain regimen or schedule. This is denoted a "question schedule" or "question regimen" in the present application. These terms are intended to be equivalent if not otherwise indicated.

The term "set of functions" means a set of functions that can be applied to the answers to a set of questions to extract specified information and generate feedback based on the answers.

The combination of a set of questions and a set of functions is referred to as a "question-feedback model".

That a set of questions is "specific" to a certain pharmaceutical product shall be construed to mean that it comprises questions that are applicable and clinically relevant to the pharmaceutical product. The individual questions, and the set of questions in total, are preferably more applicable and clinically relevant to the pharmaceutical product in question than to any other pharmaceutical product.

The term "respondent" is used to denote the individual responding to a question.

The term "patient" is used to denote the individual using the pharmaceutical product.

The terms "pharmaceutical product" and "medical product" shall be considered equivalent unless specifically indicated otherwise. These terms refer to pharmaceutically acceptable compositions of pharmaceutically active substances (drugs) intended for administration to a patient.

The term "side effect" means a secondary and usually adverse effect of a drug or treatment.

The term "adverse event" means an adverse outcome that occurs during or after the use of a drug or other intervention but is not necessarily caused by it.

### Detailed description of the invention

The main aspect of the present invention is a combination product comprising a pharmaceutical product and a computer program product comprising instructions to perform a method comprising the steps of providing a defined set of specific questions to the user, collecting answers to the questions and transforming the answers by way of a defined set of specific functions to generate feedback to the patient.

By adapting the combination of the set of questions and the set of functions, which combination is hereinafter called the "question-feedback model", to be specific to the pharmaceutical product, and optionally the therapeutic indication and/or prescribed dosage/administration regimen, it is possible to achieve an unexpected and significant improvement in the therapeutic effect of the pharmaceutical product and quality of life for patients. Without being bound by theory, the improved therapeutic effect and quality of life may be due to improved compliance by the patient to the prescribed administration and/or dosage regimen, due to improved awareness of other factors influencing the relevant condition being treated with the pharmaceutical product, or due to a placebo or placebo-like effect.

For each combination of the computer program product and the pharmaceutical product a question-feedback model is developed and adapted to the specific characteristics of the pharmaceutical product and the behavior of the patients within the actual therapeutic area(s). The development of the question-feedback model follows the same general rules for different types of pharmaceutical products, but the specific question-feedback models will be different.

The question-feedback model comprises the following parts:

A set of questions specific for the pharmaceutical product. The set of questions is implemented in a questionnaire giving the respondent the ability to choose any of a number of possible answers to each question or enter a number representing a test value. The questions may relate to the following, the list being illustrative and non-exhaustive:
- Food and drink consumption, such as meal size; meal frequency; type of diet; satisfaction with diet
- Exercise, such as type, duration, frequency or avoidance of physical exercise
- Mood, such if the respondent is happy, sad, depressed, anxious, restless, etc.
- Sleep, such as if the patient has slept well; duration or quality of sleep
- Use of tobacco, alcohol and other drugs, such as type and amount of use; urge to use; intention or inclination to quit use; progress or lack of progress in cessation
- Stress, such as perceived stress level; amount of personal quality time or spare time; amount of family quality time
- Body functions, such as function of the gastrointestinal system; mental capacity, muscle strength/weakness; olfactory capacity; cardiovascular capacity
- Symptoms, such as stiffness; swelling of limbs or joints; fatigue; dizziness; headache; pain; blood in excrement; incontinence; fever; urticaria; rashes; skin irritation; itching; anxiety; dryness of mouth or other mucosa; shortness of breath; coughing; sneezing; rhinitis; irritation; restlessness
- Treatment, such as if the treatment is perceived as working well; motivation to start or continue treatment
- Compliance to dosage and/or administration regimen, such as if or when the pharmaceutical product has been administered; or what dose was administered.
- Adverse events, such as adverse drug effects
- Quantitative test results, such as blood pressure; body fluid or excrement analysis results; body weight; Body Mass Index; pulse
- General, such as quality of life; feeling of support from family, friends, caregiver

The questions within the set preferably have a limited number of possible answers, such as yes/no; Visual Analogue Scale (VAS); Likert scale; multiple choice; etc. The questions may however also have an undefined number of answers, such as a value of a test parameter (e.g. blood pressure, blood glucose level) or free text.

Generally, the questions are posed to the patient using the pharmaceutical product because only the patient has true first hand knowledge of his or her situation. However, in addition to questions posed to the patient, further questions may be posed to other respondents. These may include family members, relatives or other persons close to the patient. This may be particularly useful for pharmaceutical products used in treatment of psychiatric disorders where the patient's assessment of his or her situation may be incomplete and observations made by another person may be valuable. Questions to be answered by other respondents may belong to the same set of questions as those answered by the patient, but may be implemented in a separate questionnaire.

The specific questions and invitations given to the respondents and the type of questions are adapted to the specific characteristics of the pharmaceutical product and the behavior of the patients within the therapeutic area in order to optimize the clinical effects.

When defining the actual questionnaire it is preferable to develop questions to the respondent in order to identify possible upcoming adverse events, or indications of adverse events, as well as possible upcoming side effects.

In addition to the set of questions, also a regimen for asking the respondent questions should be developed, including which questions are compulsory to answer, optionally before or after a certain time or within a certain time interval; which questions may be left unanswered; at what time of day the questions will show up for the respondents to answer them; with what frequency the questions shall show up etc.

In addition to the above described questions it may be advantageous to include messages that can not be answered to the respondent. Such messages may include recommendations, suggestions or information intended to motivate the respondent, e.g. to continue the precribed regimen although symptoms have disappeared or are less pronounced.

It may furthermore be advantageous to adapt the set of questions and messages and the regimen for asking the questions and providing the messages with a view to cultural differences and the language of the user. Principles for the translation and cultural adaptation process for PRO measures have been described (Wild D, et al., Value Health 2005;2:94-104) and may be adapted to the present invention by the skilled person.

The question-feedback model further comprises retrieving answers from the respondents in a predefined format suitable for input into the set of functions for generating feedback.

The question-feedback model further comprises a set of functions to generate patient-specific feedback based on the answers of the respondent or respondents. These functions may comprise:
- Calculations resulting in a realistic target for a specific patient to achieve. The target could be based on information given from the results from earlier clinical trials concerning the pharmaceutical product. The target could then, for example, be illustrated as a continuous graph of the predicted development for the patient, given that the prescribed administration or dosage regimen is followed. The illustration of this continuous graph would vary between different therapeutic areas. In some areas it would illustrate the improvement of the condition whereas in other areas, for example, COPD (Chronic Obstructive Pulmonary Disease) where patients slowly degenerates, it would illustrate the lack or relative slowness of degeneration.
- Calculations of future predictions for a specific patient, based upon earlier answers from the patient and results from clinical trials and answers from other patient in real life using the actual pharmaceutical product. These future predictions could, for example, be several predictions for each patient, based upon different circumstances in the shape of how the patient changes his/her behavior. An example of this could be if the patient increase the adherence/compliance to the specific pharmaceutical product, the patient will develop in a more positive way concerning specific symptoms for the disease.
- Knowledge and rules using, for example, methods for Computer Adaptive Testing and Item Response Theory including adapted databank with the purpose of optimal individualized and personalized medicine. This could, for example, result in an individualized questionnaire for each patient based upon their own characteristics and behavior.
- Calculation of trend lines based upon the answers given by the patient.
- Rules and thresholds for defining when to give notifications concerning different kind of issues, e.g. possible adverse events, possible side effects, change dosage regimen etc. These have to be carefully developed and take notice of possible combination between different questions, the evolvement of the answers from patients over time etc.

Patient-specific feed-back is generated by the above described set of functions based on answers supplied by the patient. The feedback may be provided through any medium favorable to the patient, e.g. through a website, a handheld device (mobile phone, PDA, etc), paper, voice, e-mail, telefax etc.

Examples of feedback are:
- Graphs illustrating the answers given by the patient on different selected questions. The graphs may, among other things, illustrate how the patient has evolved over time.
- Illustrating the answers from the patient in combination with calculated values such as targets for the patient to reach. The purpose of this type of feedback is, for instance, to motivate the patient to continuous improvements.
- Illustrations of how the patient's health status is evolving in comparison to the evolvement of earlier patients using the same pharmaceutical product, for example patients in clinical trials.
- Illustrations of how the patient's health status could evolve and the result of it as future prediction, based upon how the patient continues to handle his/her health situation. For example, graphs could be used to show how the patient might evolve if the patient increased the adherence/compliance to the medication of the pharmaceutical product.
- The, preferably de-identified, answers from the patient in relation to calculations based upon information given from other patients in real life / clinical practice using the pharmaceutical product, specifically selected for the actual circumstance. The purpose of this is, among other things, to encourage the patient to increase the personal health status.
- Message sent based upon notifications from the algorithms. This could, for example, be messages concerning possible adverse events, or indications of possible side effects, or possible conclusions that a new dosage for the actual pharmaceutical product might be needed, or positive feedback to the patient to encourage a behavior leading to e.g. better compliance or increased quality of life. Exemplary messages could include messages that the alcohol consumption is below or above a recommended threshold for the pharmaceutical product, or that the amount of consumed food is high or low in comparison to physical activity, or that the first signs of a side effect appear to be showing and that the patient should be aware of these signs. The invention will hence enable a faster change of used medicines by patients experiencing an adverse event. The patient can receive messages from the healthcare personnel as well through the computer program product, as a result of the feedback given to them.
- The questionnaire given to the patient could change based upon the algorithms for CAT and IRT (see above), or other appropriate algorithms or computer implemented methods, in order to individualize the questions for the characteristics for each patient.

Optionally, feedback may also be provided to other than the patient, such as health care staff (e.g. treating medical practitioner or nurse, pharmacist etc.). Such feedback may include: Results from notifications from the algorithms, e. g. when an adverse event or a side effect has occurred. This information could, for example, be sent to the responsible healthcare provider and/or authorities such as the Medical Product Agency. The healthcare personnel will then be able to take appropriate adjustments.
The graphs and illustrations presented above could be given to the responsible healthcare personnel as well.
Results from continuous results in real clinical trials based upon the answers given by the patients. The invention could hence improve clinical research through continuous follow up of a huge amount of patients for the specific selected pharmaceutical products. The information/answers from the patients would be de-identified and returned to the researching organization. The purpose is to utilize the enormous information in real clinical practice in order to develop improved pharmaceutical products and treatments for patients. The continuous follow-up of the results from patients will also result in possibilities for an easy evaluation between different kind of treatments, both from a medical and a economic perspective.

The question-feedback model may be adapted to the specific pharmaceutical product by using the information on the pharmaceutical product available from clinical trials carried out in preparation for an application for marketing approval for the pharmaceutical product. Such trials are designed to find all relevant information about the pharmaceutical product and that information can be used to design the set of questions with applicable answers, the set of functions for generating the feedback from the answers, and the form of feedback provided to the patient.

Information on the normal effect of the pharmaceutical product can be used to provide the patient with feedback on how he or she achieves a better or worse effect than normal when using the pharmaceutical product. It may also be used to give the patient feedback on how the treated condition would have developed if the pharmaceutical product had not been used, or used to a different extent than the patient is actually using it.

Information on known possible side effects may be used to include question giving early feedback on occurrence of side effects, which may guide the user to change or cease the administration or dosage regimen according to guidelines based on the information about side effects, or to contact the treating physician if advised.

Information on known counter-indications for using the pharmaceutical product may be used to include questions giving early feedback warning for possible adverse events. It may be that during treatment with the pharmaceutical product the patient contracts a condition which may lead to an adverse event or side effect in combination with the pharmaceutical product. If such risks are known, it is possible to include questions resulting in feedback making the patient and optionally the treating physician aware of this complication, which may lead to an adjustment or change in treatment.

For example, one specific pharmaceutical product indicated for treatment of obesity is known to worsen depressions. The majority of questions and feedback in a question-feedback model for an obesity drug would probably focus on diet, physical activity, weight loss and the like. The inclusion of one or more mood-related questions would however be able to indicate early if the patient is at risk of developing a depression which would be a strong indication to the patient to cease the administration of the pharmaceutical product. These questions should be specifically designed to retrieve relevant information on the types of mood-related adverse events or side effects associated with the specific pharmaceutical product.

Optionally, additional information not supplied directly by the patient is used. This may include
- Information from performed clinical trials. This could, for example, be the result how the included patients in the clinical trials using the actual pharmaceutical product responded to the pharmaceutical.
- Information from other patients in clinical practice. This could, for example, be the result and answers given by other patients in real life using the equivalent pharmaceutical product and how they respond to the pharmaceutical. Using that information, a common index of how a huge amount of patients react upon the actual pharmaceutical product in real life can be evaluated, for instance.
- Information from other products and systems, such as administration systems, laboratory data, personal patient devices such as watches, heart rate monitors, scales, mobile phone applications etc.

For each combination of a specific pharmaceutical product and the computer program product a candidate specific question-feedback model has to be developed. This candidate model has to be developed based on all considerations mentioned above.

The development of the candidate question-feedback model includes the following steps:

An optimal set of questions is identified and developed. In this, the following aspects should be considered, as well as the concerns mentioned above describing what is included in the set of questions.
- The set of questions should be designed based upon the specific circumstances of the pharmaceutical product concerning the existence of possible adverse events, possible side effects and the therapeutic effect.
- The set of questions should be designed based upon the special circumstances of the patient category of the actual therapeutic area.
- The set of questions should be designed in order to optimize the behavioral aspects of the patients. They should increase the possibilities for enhanced quality of life for the patients.
- The questions should be easy to understand and encourage the patient to answer them. The suitable and optimal structure type of questions should be used, i.e. VAS, Likert scale, free text etc.
- The amount of questions should be minimized in order to simplify for the patients.
- The proper regimen for asking the respondent questions should be developed. The following should, for example, be defined:
   o When the questions should appear in the patient's device, for instance which specific day and what time during the day
   o Which questions that should be compulsory to answer
   o The frequency of how often the questions should appear in the patient's device
   o Which questions that should be able to individualize, i.e. to add or remove, and to which extent. For example, some questions could be able to appear more or less seldom, i.e. changing the frequency of the question.

An optimal set of functions is identified and developed. In this, the following aspects should be considered, as well as the concerns mentioned above describing what is included in the set of functions.
- The set of functions should be designed based upon the specific circumstances of the pharmaceutical product concerning the existence of possible adverse events, possible side effects and the therapeutic effect.
- The set of functions should be designed based upon the special circumstances of the patient category of the actual therapeutic area.
- The set of functions should be designed in order to optimize the behavioral aspects of the patients. They should increase the possibilities for enhanced quality of life for the patients.
- The set of functions should be developed based upon which type of information that is possible to use considering the specific pharmaceutical product, e.g. if there are information from earlier clinical trials and/or if information from other patients in clinical practice, that can be utilized.
- The set of functions should be developed based upon whether knowledge and rules from methods using Item Response Theory and Computer Adaptive Testing, or other appropriate algorithms or computer implemented methods, are available.
- The set of functions concerning rules and thresholds, for example with the purpose of avoiding possible adverse events and/or side effects, giving positive feedback and optimizing the dosage regimen, should be developed concerning the circumstances of the pharmaceutical product and the specific patient population.

An optimal type of feedback should be identified and developed. In this, the following aspects should be considered, as well as the concerns mentioned above describing what is included in the type of feedback.
- The type of feedback should be designed based upon the specific circumstances of the pharmaceutical product concerning the existence of possible adverse events, possible side effects and the therapeutic effect.
- The type of feedback should be designed based upon the special circumstances of the patient category of the actual therapeutic area.
- The type of feedback should be designed in order to optimize the behavioral aspects of the patients. They should increase the possibilities for enhanced quality of life for the patients.
- It should be defined which type of feedback that should be given to whom.
- The type of feedback should be designed and developed based upon whom to which the feedback should be given to.
- The type of feedback should be designed and developed based upon the developed set of questions and set of functions for the specific question-feedback model.

It may be desirable to furthermore optimize the set of questions and the feedback for use on a certain computer platform. For instance, if the respondent will use a simple mobile telephone the questions will be adapted so that they can be answered simply by pressing buttons 0-9 and yes/no/up/down and feed back may be provided in short text messages and simple graphs. If the respondent uses an advanced mobile telephone the questions may be constructed to give more complex answers and still be easy to use, and the feedback may also be made more complex, such as colour-coded graphs and longer messages.

The candidate question-feedback model is then validated in one or more steps. The validation of the model aims to evaluate the therapeutic effect of the integrated combination of the computer program product and pharmaceutical product, minimize the amount of adverse events and side effects, and increase the quality of life for the patients. The evaluation of the clinical efficacy and value of the candidate question-feedback model for a specific pharmaceutical product is preferably performed through clinical trials, in what is usually referred to as a Phase II clinical trial. In this the candidate question-feedback model for the pharmaceutical product is evaluated concerning clinical efficacy such as positive medical efficacy and increased security level for the combination product.

There are a number of types and designs of clinical trials and a skilled person would be able to choose a type of trial and design well suited to achieve the aims as outlined herein. The clinical trials should be designed to focus to prove the following of the model enabling the combination of the computer program product and the pharmaceutical product:
- achieve optimum medical efficacy of the combined product
- achieve optimum level of safety for patients
- increase quality of life for the patient

Based on progress and results from clinical trials, the question-feedback model may of course be adjusted or revised in order to improve its efficacy, safety or other aspects of quality.

The combination of the question-feedback model and the pharmaceutical product may also be compared to an existing approved treatment in a Phase III-type clinical trial before being put on the market.

The question-feedback model is implemented in one or more computer-program products running on one or more computer platforms, wherein the computer program product and the computer platform together have means for providing the set of questions, for receiving the answers, for applying the set of functions to generate the patient-specific feedback and preferably also for providing said feedback to the patient.

The computer program product may be supplied on a suitable carrier together with the pharmaceutical product, as a kit-of-parts. Suitable carriers are well-known to the skilled person and depend on the platform on which the computer program product shall run, but includes without limitation, CD-ROM, USB-memory sticks, flash memory cards. The computer program product may also be made available to the end user separately from the physical pharmaceutical product. This can be done e.g. by supplying information on how to access the computer program product on a remote server and install the computer program product on the relevant platform with the pharmaceutical product. The computer program product may be included in the kit-of-parts in the form of instructions for accessing and/or installing the computer program product from a remote location, such as a remote server. Information about how to get started with the computer program product and how to use it could be given in the instructions related to the pharmaceutical product or the computer program product.

If the computer program product is made available separately from the pharmaceutical product, a unique identifier may be provided with each individual kit. The identifier may be used to confirm that the respondent has got the correct combination of computer program product and pharmaceutical product and to confirm that the respondent has the right to use the computer program product.

The computer program product is an essential part of the main aspect of the invention and is itself one aspect of the invention, as is the method implemented in the computer program product.

The pharmaceutical product may be any pharmaceutical product for which there exists a preferred or prescribed administration and/or dosage regimen. This includes all pharmaceutical products that have been approved for marketing based on results of clinical trials defining a therapeutically effective dose or dose range and pharmaceutical products for which a medical or other practitioner prescribes an individual administration or dosage regimen to an individual patient based on information supplied by the manufacturer of the pharmaceutical product. It furthermore includes pharmaceutical products for which an application for marketing approval is to be submitted, pending or have been refused. The pharmaceutical product may or may not be subject to regulation by a Medical Products Agency or other governmental agency, it may be a prescription only product, an over-the-counter product or any other allegedly therapeutically active product, such as a herbal medicinal product.

Examples of pharmaceutical products that can be used in the present invention are Abilify Acomplia, Actos, Glucosine, Advate, Advair, Seretide, Ambien, Stilnox, Zolpidem, Apidra, Aricept, Avapro, Aprovel, Avandia, Avandamet, Avandaryl, Avastin, Avonex, Aranesp, Arimidex, Atacand, Benicar, Olmetec, Betaseron, Betaferon, Byetta, Casodex, Celebrex, Celebra, Cipralex/Lexapro, Cymbalta, Champix, Copaxone, Coreg, Cozaar, Crestor, Tritace, Diovan, Efexor, Eloxatin, Eloxatine, Enbrel, Evista, Ezetrol, Zetia, Flomax, Flomaxtra, Urimax, Flovent, Flixotide, Fosamax, Gemzar, Gleevec, Glivec, Herceptin, Humalog, Humira, Kaletra, Imitrex, Imigran, Januvia, Lantus, Lipanthyl, TriCor, Lipitor, Levemir, Lotrel, Leuprorelin, Lupron, Leuplin, Lyrica, Mabthera, Rituxan, Micardis, Nexium, Norvasc, NovoLog, NovoMix, NovoRapid, NovoNorm, Pariet, Paxil, Seroxat, Protonix, Pantozol, Pantoloc, Plavix, Pravachol, Procrit, Protopic, Eprex, Pulmicort, Rebif, Reductil, Remicade, Risperdal, Seloken, Toprol, Seroquel, Spiriva, Symbicort, Singulair, Taxotere, Topamax, Truvada, Vytorin, Wellbutrin, Xamiol Taclonex, Zocor (simvastatin), Zoloft (Sertralin), Zometa, Zyprexa, Zyrtec, Cetirizine

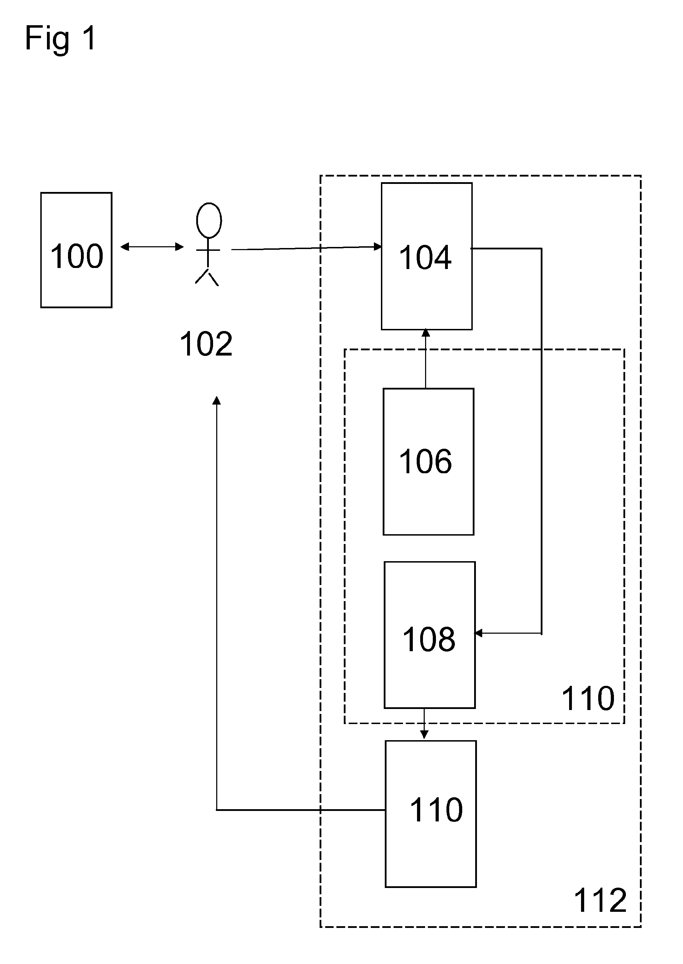

The invention will now be described in relation to the appended drawings. Figure 1 shows a pharmaceutical product (100) available to a patient/respondent (102). A set of questions (106) and a set of functions (108) for converting the answers to the questions into patient feedback are implemented in a computer program product (110) running on a computer platform (112) having means (104) for receiving answers to said set of questions from said patient. The computer platform further has means (108) for receiving patient feedback from the set of functions (106) and communicating said feedback to said patient (102).

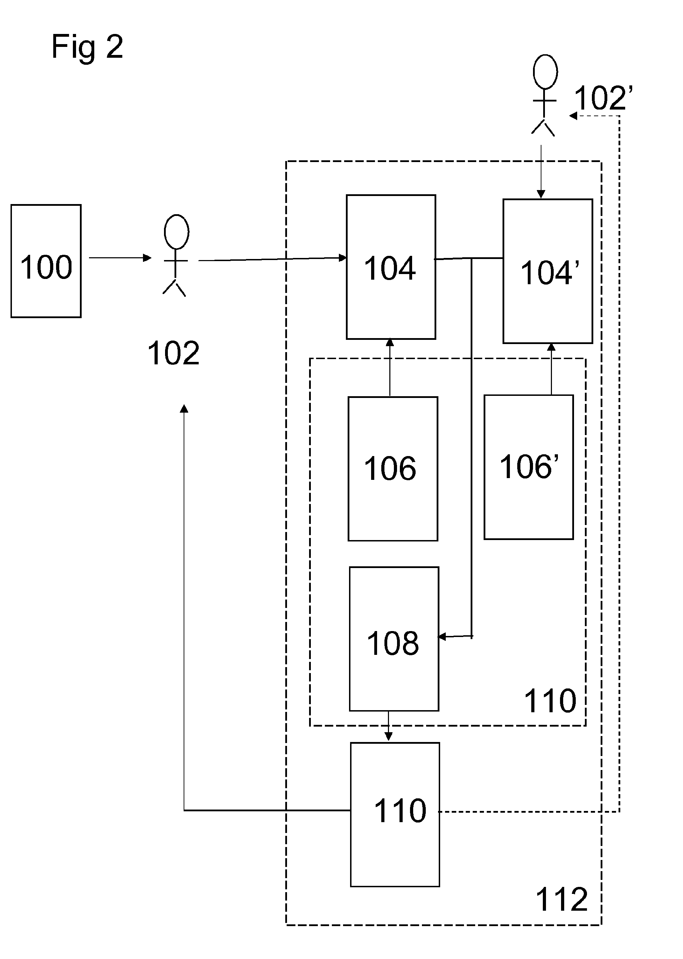

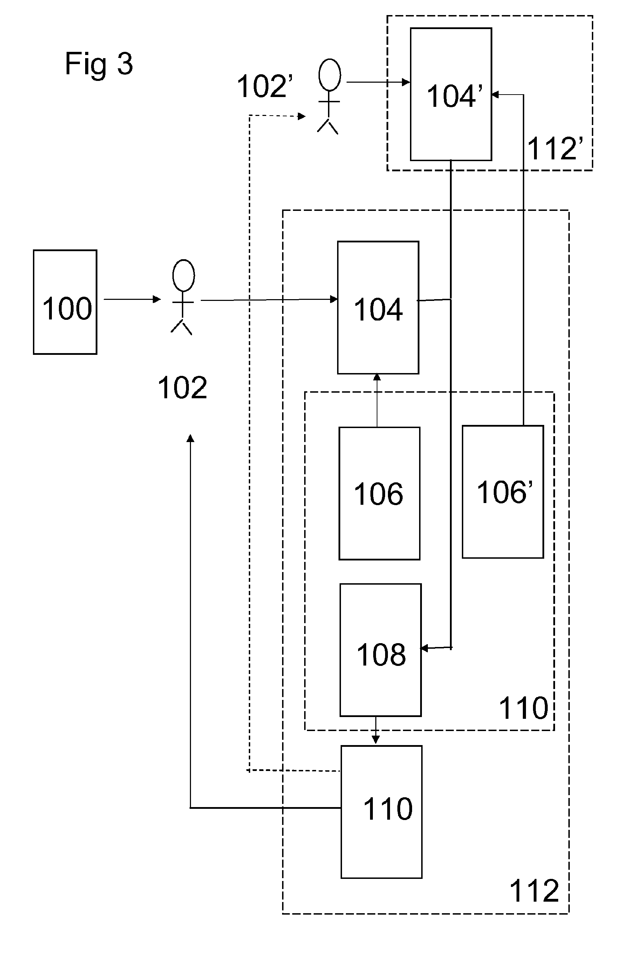

Figure 2 shows an alternative embodiment of the invention, wherein a further respondent (102') answers a second set of questions (106') through means (104') for receiving answers to said set of questions from said further respondent. The answers to the set (106') is then provided together with the answers to the set (106) to the set of functions (108) to generate feedback to patient (102). Optionally, feedback is also provided to the further respondent (102'), shown with a dotted line. The further respondent may be a person close to the patient, such as a family member. The means (104') for receiving answers from the further respondent may be implemented on a separate computer platform (112'), cf Figure 3.

The examples below serve to further illustrate the invention, provide experimental support and enable the skilled person to work the invention. They shall not be construed as limiting the scope of the invention, which is that defined by the appended claims.

### Example 1, Type II Diabetes and Lantus

A study is performed as described below to substantiate the efficacy of the invention. The study shall comply with national and international rules, legislation and practices with regards to e.g. ethics, informed consent, protection of confidential personal information, reporting of adverse events.
Study length: 12 months.
Design: Open controlled study.
Population: Men and women with elevated HbA1c (>8%).
Number of patients: 300 receiving intervention, 300 control.
Studied treatment: Combination of using the interactive communication tool, including receiving active feedback, and Lantus(TM), the prescribed pharmaceutical product, in accordance with the invention, during the study period.
Primary aim: To evaluate change in HbA1c before and after 12 months of the studied treatment.
Secondary aims: Concerning the studied treatment: Evaluate the change in the amount of adverse events and side effects; Evaluate effect on blood pressure and blood lipids; evaluate other variables routinely collected by medical staff; Evaluate effects of treatment on quality of life (SF-36); Evaluate the patient's perceived benefit from treatment; Evaluate medical staff's perceived benefit from treatment.
Evaluate adherence/compliance to Lantus.
Examinations: Evaluation according to standard practice.
Sampling: Sampling according to standard practice.
Treatment information: Transfer of information related to study treatment to and from the patient: effect, adherence, symptom, adverse events, and knowledge about treatment.

Communication tool: The invention is implemented in the framework of the communication tool CQ (CircadianQ, Stockholm, Sweden). CQ (Circadian Questions) is an interactive mobile follow-up service that can be used i.e. in follow-up or support in medical treatment. CQ runs on the respondent's own mobile phone and the set of questions are presented by the mobile phone at predefined points in time. Structured answers can be given through VAS (Visual Analogue Scale), which are easy to answer and gives illustrative graphs. Questions relating to multiple choices, integers and decimal numbers may also be posed, and encouragement, recommendations and information may be provided. The questions relating to physical exercise have been tested in previous studies and shown to have a good correspondence between patients' answers and actual performed exercise, according to the doubly labeled water method (Bexelius C, et al., J Med Internet Res 2010, 12(1):e2).

The goal of the study is to substantiate that the use of the invention can:
- Improve the therapeutic effect of Lantus in clinical practise
- Decrease the amount of adverse events and side effects
- Increase amount of physical exercise;
- Improve dietary habits;
- Improve the perceived quality of life/wellbeing before and after the study.
- Improve adherence/compliance to Lantus

The study project is an open randomised study. The project comprises a total of 300 patients that will be offered access to CQ with a specific question-feedback model. A control group is randomly chosen to be followed and receive only standard treatment and support from nurses. The patients will continuously during 12 months receive questions, support and information through their mobile phones in accordance with the set of questions specified below. The answers are registered in a database, from where the set of functions will operate in order to create feedback.

The feedback to the patients will consist of the following components:
- Graphs based upon the answers from the patient. Visualizing in a structured manner correlations between different questions, e.g. the result on health status when the patient is adherent to the prescribed regimen of Lantus. In the graphs the answers from the patient will, for instance, be visualized over time.
- The graphs will be illustrated as well using trend lines. These will appear when the patient has been answering questions for more than a month.
- The patient's evolvement over time is illustrated, for instance in graphs, in relation to realistic health targets. The health targets, which are developed for specific questions, are based upon data from earlier clinical studies/trials performed on Lantus. The targets will be individualized for each patient, depending on the amount of available information.
- The patient will be sent short messages based upon their health evolvement. These messages will be sent as encouraging and motivating information when the patient:
   o Has fulfilled something positive, such as been adherent to Lantus and/or accomplished a stable proper P-glucose value.
   o Needs a "small push" in order to improve their behaviour, for example to become more adherent to Lantus.
- In relation to each patient's evolvement and the answers, future predictions concerning his/her health status will be developed. The future predictions will visualize possible scenarios for the patient based upon how the patient will evolve in some critical issues, for example the compliance/adherence to Lantus in relation to health status and level of wellbeing.
- The set of functions concerning possible adverse events and side effects for Lantus will be implemented. The possible adverse events and/or side effects will be visualized for the patient depending on the safety concern. Possible adverse events will be sent by messages and possible side effects will be visualized in context with the given answers, for example in different graphs or other illustrations. The possible adverse events will be sent to health responsible as well.

The patients will be recommended to adjust their dose of Lantus if necessary.

Patients at the diabetes practices with an Hb1Ac > 8% are informed about the study and the possibility to enter the study. Patients willing to participate are consecutively included. Patients will receive a short introduction of the communication tool. If necessary, patients will be able to contact technical support for the tool. If a patient included by medical staff does not start using the tool, the tool will automatically contact the patient to offer technical support, subject to approval of the patient.

The patients then answer the questions according to a predefined schedule during the course of the study or until the patient chooses to abort participation. The questionnaire regimen is possible to individualize, which is, for example, to remove some questions and to change the specific time when they will show up on the patient's mobile phone. Patients are informed that they shall contact medical staff if they become acutely ill or if their condition seriously deteriorates.

At the beginning the patient fills out a questionnaire relating to basic facts about his or her personal situation and health situation, his or her commitment to treatment, perceived commitment from medical staff, quality of life, consent to staff to retrieve data from the patient's medical records and to participate in the study, etc. Weight, length, waist-measure and HbA1c is measured and recorded at inclusion in the study and after 3 months. After 12 months the same questions are asked again and the intra-individual change is calculated.

Criteria for inclusion:
- Diabetes with HbA1c > 8%
- Access to a mobile telephone
- Ability to follow the CQ programme

Criteria for exclusion:
- Patients who cannot read and/or understand the principal language of the study
- Patients with severely reduced cognitive abilities
- Patients with reduced vision leading to an inability to answer the questions

### Starting set of questions and question schedule, Lantus

Questions, preferably during the evening, every second day month 1-2, every fourth day month 3-4, once per week month 5-12.
1. What was your P-glucose value this morning?
   (numeric)
2. Have you adjusted your insulin dose based on the P-glucose value?
   (yes / no)
3. Have you taken all insulin doses as planned during the day?
   (yes completely / yes partially / no)
4. Physical exercise today?
   (no / light <30 min / medium >30 min / heavy > 60 min)
5. Do you take Lantus according to the prescription?
   (yes / partially / no)
6. How was your injection of Lantus today?
   (VAS; 0=Extremely poor, 10=Extremely good)
7. Have you had any of the following symptoms of low blood sugar recently?
   (headache / nausea / hunger / confusion / drowsiness / weakness / dizziness / blurred vision / fast heartbeat / sweating / tremor / trouble concentrating)

Questions, preferably during the evening, every second day during the two first weeks, once per week during month 4 and once per week during month 8
1. Have you felt something of the following?
   (widespread rash or itchiness / wheezing / trouble breathing / fast heart rate / swelling of the mouth, face, lips, or tongue /sweating / feeling like you might pass out)
2. Do you have the following signs on your skin where you inject Lantus?
   (itching / swelling / redness / thickening)

Questions once per week month 1-3, once every two weeks month 4-9, once per week month 10-12.
3. How have you slept this week?
   (VAS; 0=Extremely poor, 10=Extremely good)
4. How much have you been exercising this week, compared to your maximum capability?
   (VAS; 0=Nothing at all, 10=At my maximal capacity)
5. To what extent has your diabetes affected your activities this week?
   (VAS; 0=Not at all, 10=To a very large extent)
6. Your weight this morning?
   (numeric)
7. Your quality of life as regards to health?
   (VAS; 0=extremely bad QoL, 10=extremely good QoL)

Questions every second week month 1-4, once per month during month 5-12.
1. Do you vary the place of your injection of Lantus?
   (VAS; 0=Not at all, 10=Every time)
2. Has your Lantus been anything else than thin, clear, and colourless during the last two weeks?
   (yes / partially / no)

Questions once per month, month 1-12
1. Number of standard measures of alcohol last 24 hours?
   (numeric)
2. Portion size of breakfast today?
   (VAS; 0=Very small, 10=Very large)
3. Cooked meal for lunch today?
   (yes / no)
4. Portion size of lunch today?
   (VAS; 0=Very small, 10=Very large)
5. Portion size of dinner today?
   (VAS; 0=Very small, 10=Very large)
6. How is the practical handling of Lantus, including the injection pen, working out for you?
   (VAS; 0=Extremely bad, 10=Extremely good)
7. Have you, during the last month, been taking any of the following medications as well?
   (albuterol (e.g. Proventil, Ventolin) / clonidine (e.g. Catapres) / reserpine / guanethidine (e.g. Ismelin) / a beta-blocker)

Questions once per month, month 1-12 (other day than questions above)
1. How do you feel?
   (VAS; 0=Extremely bad, 10=Extremely good)
2. Are you experiencing stress right now?
   (VAS; 0=Not at all, 10=Extremely much)
3. Did you feel well rested this morning?
   (VAS; 0=Not at all rested, 10=Extremely well rested)
4. Are you satisfied with your treatment?
   (VAS; 0=Not at all satisfied, 10=Extremely satisfied)
5. Are you satisfied with the forms of exercise you perform?
   (VAS; 0=Not at all satisfied, 10=Extremely satisfied)
6. Are you satisfied with your diet?
   (VAS; 0=Not at all satisfied, 10=Extremely satisfied)

Spontaneous questions, which could be answered by the patients, whenever they wish:
1. How do you feel?
   (VAS; 0=Extremely bad, 10=Extremely good)
2. Have you had any of the following symptoms of low blood sugar recently?
   (headache / nausea / hunger / confusion / drowsiness / weakness / dizziness / blurred vision / fast heartbeat / sweating / tremor / trouble concentrating)

### Example 2, General Anxiety Disorder, Lyrica

A study is performed as described below to substantiate the efficacy of the invention. The study shall comply with national and international rules, legislation and practices with regards to e.g. ethics, informed consent, protection of confidential personal information, reporting of adverse events.
Study length: 4 months.
Design: Open controlled study.
Population: Men and women with General Anxiety Disorder
Number of patients: 300 receiving intervention, 300 control.
Studied treatment: Combination of using the interactive communication tool, including receiving active feedback, and Lyrica(TM), the prescribed pharmaceutical product, in accordance with the invention, during the study period.
Primary aim: To evaluate change in the level of anxiety before and after 4 months of the studied treatment.
Secondary aims: Concerning the studied treatment: Evaluate effect on level of fatigue; Evaluate effect on level of muscle tension; Evaluate effect on level of depression; Evaluate other variables routinely collected by medical staff; Evaluate effect on quality of life (SF-36); Evaluate the patient's perceived benefit from treatment; Evaluate medical staff's perceived benefit from treatment; Evaluate the amount of adverse events; Evaluate adherence/compliance to Lyrica.
Examinations: Evaluation according to standard practice.
Sampling: Sampling according to standard practice.
Treatment information: Transfer of information related to study treatment to and from the patient: effect, adherence, symptom, adverse events, and knowledge about treatment.

Communication tool: The invention is implemented in the framework of the communication tool CQ (CircadianQ, Stockholm, Sweden). CQ (Circadian Questions) is an interactive mobile follow-up service that can be used i.a. in follow-up or support in medical treatment. CQ runs on the respondent's own mobile phone and the set of questions are presented by the mobile phone at predefined points in time. Structured answers can be given through VAS (Visual Analogue Scale), which are easy to answer and gives illustrative graphs. Questions relating to multiple choices, integers and decimal numbers may also be posed, and encouragement, recommendations and information may be provided.

The goal of the study is to substantiate that the use of the invention can:
- Increase therapeutic clinical effect of Lyrica;
- Decrease the amount of adverse events for the patients during the study period
- Improve target compliance for risk factors for general anxiety disorder
- Identify perceived quality of life/wellbeing before and after the study.
- Improve adherence/compliance to Lyrica

The study project is an open randomised study. The project comprises a total of 300 patients that will be offered access to CQ with a specific question-feedback model. A control group is randomly chosen to be followed and receive only standard treatment and support from nurses. The patients will continuously during 4 months receive questions, support and information through their mobile phones in accordance with the set of questions specified below. The answers are registered in a database, from where the set of functions will operate in order to create feedback.

The feedback will consist of the following components:
- Graphs based upon the answers from the patient. Visualizing in a structured manner correlations between different questions, e.g. the result on health status when the patient is adherent to the prescribed regimen of Lyrica. In the graphs the answers from the patient will, for instance, be visualized over time.
- The graphs will be illustrated as well using trend lines. These will appear when the patient has been answering questions for more than a month.
- The patient's evolvement over time is illustrated, for instance in graphs, in relation to realistic health targets. The health targets, which are developed for specific questions, are based upon data from earlier clinical studies/trials performed using Lyrica. The targets could be individualized for each patient, depending on the amount of available information.
- The patient will be sent short messages based upon his/her health evolvement. These messages will be sent as encouraging and motivating information when the patient:
   o Has fulfilled something positive, such as been adherent to Lyrica or achieved a stable and positive level in questions concerning worry, sleep and/or muscle tension.
   o Needs positive feedback in order to improve their behaviour, for example to become more adherent to Lyrica.
- In relation to each patient's evolvement and the given answers, future predictions concerning his/her health status will be developed. The future predictions will visualize possible scenarios for the patient based upon how the patient will evolve in some critical issues, for example the compliance/adherence to Lyrica in relation to health status and level of wellbeing.
- The set of functions concerning possible adverse events and side effects for Lyrica will be implemented. The possible adverse events and/or side effects are visualized for the patient depending on the safety concern. Possible adverse events will be sent by messages and possible side effects will be visualized in context with the given answers, for example in different graphs or other illustrations. The possible adverse events will be sent to health responsible as well.
- Each patient will be presented the average evolvement for other patients in similar situation as themselves. This information will be fully de-identified and will be presented in parallel with the patient's own development. The purpose is to encourage the patient to certain change in behaviour, for example to stay adherent to Lyrica, in order to increase the level of wellbeing and other variables such as improve the results concerning the primary and secondary aims of the study.
- The feedback will be possible to individualize, i.e. to remove information if that seems motivated.

The patients will be recommended to adjust their dose to Lyrica if necessary.

Patients at the clinical practices with general anxiety disorder are informed about the study and the possibility to enter the study. Patients willing to participate are consecutively included. Patients will receive a short introduction of the communication tool. If necessary, patients will be able to contact technical support for the tool. If a patient included by medical staff does not start using the tool, the tool will automatically contact the patient to offer technical support, subject to approval of the patient.

The patients then answer the questions according to a predefined schedule during the course of the study or until the patient chooses to abort participation. The questionnaire regimen is possible to individualize, which is, for example, to remove some questions and to change the specific time when they will show up on the patient's mobile phone. Patients are informed that they shall contact medical staff if they become acutely ill or if their condition seriously deteriorates.

At the beginning the patient fills out a questionnaire relating to basic facts about his or her personal situation and health situation, facts about the primary and secondary aims of the study, quality of life, consent to staff to retrieve data from the patient's medical records and to participate in the study, etc. After 4 months the same questions are asked again and the intra-individual change is calculated.

Criteria for inclusion:
- Patients with general anxiety disorder
- Access to a mobile telephone
- Ability to follow the CQ programme
- > 18 years old

Criteria for exclusion:
- Patients who cannot read and/or understand the principal language of the study
- Patients with severely reduced cognitive abilities
- Patients with reduced vision leading to an inability to answer the questions

### Starting set of questions and question schedule, Lyrica

Questions; every second morning during month 1 and 2, and one morning per week month 3-4.
1. Have you been worried during the night?
   (VAS; 0=Not at all worried, 10=Extremely worried)
2. How did you sleep this night?
   (VAS; 0=Extremely bad, 10=Extremely good)
3. How often did you wake up during the night?
   (numeric)
4. Did you have difficulties in falling asleep?
   (yes / no)
5. Have you had muscle tensions during the night?
   (VAS; 0=None, 10=Extremely much)
6. Do you take your Lyrica according to the prescription?
   (yes / partially / no)

Questions; every second evening during month 1 and 2, and one evening per week month 3-4.
1. Have you been worried during the day?
   (VAS; 0=Not at all worried, 10=Extremely worried)
2. Have you been able to perform your everyday activities today?
   (yes / into some extent / not really / no)
3. Have you been exercising today?
   (VAS; 0=Not at all, 10=Extremely much)
4. Have you had muscle tensions today?
   (VAS; 0=None, 10=Extremely much)
5. Do you take your Lyrica according to the prescription?
   (yes / partially / no)
6. Have you, during the day, felt something of the following?
   (dizziness / indisposition / irritability / fatigue / other symptom)
7. How do you feel?
   (VAS; 0=Extremely bad, 10=Extremely good)

Questions; every second evening during the first week.
1. Have you, during the last two days, felt something of the following?
   (heart failure / kidney disease)

Questions; every second evening during the first two weeks.
1. Have you, during the last two days, felt something of the following?
   (difficulty breathing / swelling of your face, lips, tongue, or throat)

Questions once a week during month 1 and 2, every second week during month 3 and 4:
1. Your quality of life as regards to health?
   (VAS; 0=Extremely bad QoL, 10=Extremely good QoL)
2. Your weight today?
   (numeric)
3. Have you, during the last week, felt something of the following?
   (mood or behaviour changes / depression / anxiety / insomnia / feel agitated / hostile / restless / hyperactive (mentally or physically) / have thoughts about suicide or hurting yourself)
4. Have you, during the last week, felt something of the following serious side effects while using Lyrica?
   (muscle pain, weakness, or tenderness (especially if you also have a fever and feel tired) / easy bruising or bleeding / swelling in your hands or feet)
5. Remember to keep taking Lyrica even if you feel fine.
6. Do you take any of the following medication as well, which could interact with Lyrica?
   (heart or blood pressure medication e.g. benazepril / allergy medicine / sedatives / narcotic pain medicine / sleeping pills / muscle relaxers / medicine for depression or anxiety)

Questions once a month during month 1 to 4:
1. Your quality of life as regards to health?
   (VAS; 0=Extremely bad QoL, 10=Extremely good QoL)
2. Have you been in contact with another healthcare professional during the last month?
   (yes / no)
3. Have you, during the last month, felt something of the following?
   (visual disturbance / memory impairment / dry mouth / tremor / erectile dysfunction)

Spontaneous questions, which could be answered by the patient whenever he/she wish:
1. How do you feel?
   (VAS; 0=Extremely bad, 10=Extremely good)
2. Do you feel any of the following?
   (dizziness / indisposition / irritability / fatigue)
3. Have you, during the last week, felt something of the following serious side effects while using Lyrica?
   (muscle pain, weakness, or tenderness (especially if you also have a fever and feel tired) / easy bruising or bleeding / swelling in your hands or feet)

### Example 3, Dermatological diseases, Protopic

A study is performed as described below to substantiate the efficacy of the invention. The study shall comply with national and international rules, legislation and practices with regards to e.g. ethics, informed consent, protection of confidential personal information, reporting of adverse events.
Study length: 3 months.
Design: Open controlled study.
Population: Men and women with Atopic dermatitis
Number of patients: 300 receiving intervention, 300 control.
Studied treatment: Combination of using the interactive communication tool, including receiving active feedback, and Protopic(TM) in accordance with the invention, during the study period.
Primary aim: To evaluate change in level of visible rash before and after 3 months of the studied treatment.
Secondary aims: Concerning the studied treatment: Evaluate change in level of itchiness; Evaluate other variables routinely collected by medical staff; Evaluate effect on quality of life (SF-36); Evaluate the patient's perceived benefit from treatment; Evaluate medical staff's perceived benefit from treatment; Evaluate adherence/compliance to Protopic. Examinations: Evaluation according to standard practice.
Sampling: Sampling according to standard practice.
Treatment information: Transfer of information related to study treatment to and from the patient: effect, adherence, symptom, adverse events, and knowledge about treatment.

Communication tool: The invention is implemented in the framework of the communication tool CQ (CircadianQ, Stockholm, Sweden). CQ (Circadian Questions) is an interactive mobile follow-up service that can be used, i.e. in follow-up or support in medical treatment. CQ runs on the respondent's own mobile phone and the set of questions are presented by the mobile phone at predefined points in time. Structured answers can be given through VAS (Visual Analogue Scale), which are easy to answer and gives illustrative graphs. Questions relating to multiple choices, integers and decimal numbers may also be posed, and encouragement, recommendations and information may be provided.

The goal of the study is to substantiate that the use of the invention can:
- Increase therapeutic effect of Protopic in clinical practise;
- Decrease the amount of adverse events and side effects for the patients during the study period
- Increase perceived quality of life before and after the study.
- Improve patient's sleep during night
- Improve adherence/compliance to Protopic

The study project is an open randomised study. The project comprises a total of 300 patients that will be offered access to CQ with a specific question-feedback model. A control group is randomly chosen to be followed and receive only standard treatment and support from nurses. The patients will continuously during 3 months receive questions, support and information through their mobile phones in accordance with the set of questions specified below. The answers are registered in a database from where the set of functions will operate in order to create feedback.

The feedback will consist of the following components:
- Graphs based upon the answers from the patient. Visualizing in a structured manner correlations between different questions, e.g. the result on health status when the patient is adherent to the prescribed regimen of Protopic. In the graphs the answers from the patient will, for instance, be visualized over time.
- The graphs will be illustrated as well using trend lines. These will appear when the patient has been answering questions for more than a month.
- The patient's evolvement over time is illustrated, for instance in graphs, in relation to realistic health targets. The health targets, which are developed for specific questions, are based upon data from earlier clinical studies/trials performed using Protopic. The targets could be individualized for each patient, depending on the amount of available information.
- The patient will be sent short messages based upon his/her health evolvement. These messages will be sent as encouraging and motivating information when the patient:
   o Has fulfilled something positive, such as been adherent to Protopic or achieved a stable and positive level of rash or level of itchiness.
   o Needs positive feedback in order to improve their behaviour, for example to become more adherent to Protopic.
- The set of functions concerning possible adverse events and side effects for Protopic will be implemented. The possible adverse events and/or side effects are visualized for the patient depending on the safety concern. Possible adverse events will be sent by messages and possible side effects will be visualized in context with the given answers, for example in different graphs or other illustrations. The possible adverse events will be sent to health responsible as well.

The patients will be recommended to adjust their dose of Protopic if necessary.

Patients at the clinical practice with atopic dermatitis are informed about the study and the possibility to enter the study. Patients willing to participate are consecutively included. Patients will receive a short introduction of the communication tool. If necessary, patients will be able to contact technical support for the tool. If a patient included by medical staff does not start using the tool, the tool will automatically contact the patient to offer technical support, subject to approval of the patient.

The patients then answer the questions according to a predefined schedule during the course of the study or until the patient chooses to abort participation. The questionnaire regimen is possible to individualize, which is, for example, to remove some questions and to change the specific time when they will show up on the patient's mobile phone. Patients are informed that they shall contact medical staff if they become acutely ill or if their condition seriously deteriorates.

At the beginning the patient fills out a questionnaire relating to basic facts about his or her personal situation and health situation, facts about the primary and secondary aims of the study, quality of life, consent to staff to retrieve data from the patient's medical records and to participate in the study, etc. After 3 months the same questions are asked again and the intra-individual change is calculated.

Criteria for inclusion:
- Atopic dermatitis
- Access to a mobile telephone
- Ability to follow the CQ programme
- >18 years

Criteria for exclusion:
- Patients who cannot read and/or understand the principal language of the study
- Patients with severely reduced cognitive abilities
- Patients with reduced vision leading to an inability to answer the questions

### Starting set of questions and question schedule, Protopic

### Questions, every second day during the study period

1. How much eczema do you have?
   (VAS; 0=Nothing, 10=Extremely much)
2. How much itching have you had during the last 24 hours?
   (VAS; 0=Nothing, 10=Extremely much)
3. How often do you use Protopic?
   (Every day / Every second day / Twice a week / Not at all)
4. From a practical point of view - how is Protopic working for you?
   (VAS; 0=Extremely bad, 10=Extremely good)
5. Have you been able to perform your everyday activities today?
   (yes / into some extent / not really / no)

Question during the first week, every second day (same as above).
1. Have you, during the last two days, felt something of the following?
   (burning / stinging / tingling / soreness of treated skin)

Questions once per week during the study period
1. How have you been sleeping during night this week?
   (VAS; 0=Extremely poor, 10=Extremely good)
2. Do you use some of the following products as well?
   (Cortisol / moisturizing cream or lotion)
3. To what extent has your diagnose affected your activities this week?
   (VAS; 0=Not at all, 10=To a very large extent)
4. Your quality of life as regards to health?
   (VAS; 0=Extremely bad QoL, 10=Extremely good QoL)

Questions twice a week during the study period
1. Have you, during the last week; felt something of the following signs of allergic reactions to Protopic?
   (hives / difficulty breathing / swelling of your face, lips, tongue, or throat)
2. Have you, during the last week, felt something of the following Protopic side effects?
   (worsened skin symptoms / signs of a skin infection (redness, swelling, itching, oozing) / fever, chills, body aches, flu symptoms / swollen glands)
3. Have you, during the last two weeks, felt something of the following less serious Protopic side effects?
   (swollen hair follicles / acne / upset stomach / muscle pain / headache)
4. Remember to avoid sunlight, sun lamps, tanning beds, and phototherapy treatments while using Protopic.
5. There may be other drugs or skin products that can affect Protopic. Do you use any of the following medications?
   (vitamins / minerals / herbal products / cimetidine (Tagamet) / erythromycin (e.g. Ery-Tab) / antifungal medicine such as ketoconazole / heart medicine)

Spontaneous questions, which could be answered by the patient whenever the wish:
1. How do you feel?
   (VAS; 0=Extremely bad, 10=Extremely good)
2. Have you, during the last week, felt something of the following?
   (hives / difficulty breathing / swelling of your face, lips, tongue, or throat)
3. Have you, during the last week, felt something of the following Protopic side effects?
   (worsened skin symptoms / signs of a skin infection (redness, swelling, itching, oozing) / fever, chills, body aches, flu symptoms / swollen glands)

### Example 4, Hypertension

A study is performed as described below to substantiate the efficacy of the invention. The study shall comply with national and international rules, legislation and practices with regards to e.g. ethics, informed consent, protection of confidential personal information, reporting of adverse events.
Study length: 6 months.
Design: Open controlled study.
Population: Men and women with hypertension
Number of patients: 400 receiving intervention, 400 control.
Studied treatment: Combination of using the interactive communication tool, including receiving active feedback, and Atacand(TM) prescribed in accordance with the invention, during the study period.
Primary aim: To evaluate the change in blood pressure before and after 6 months of the studied treatment.
Secondary aims: Concerning the studied treatment: Evaluate other variables routinely collected by medical staff; Evaluate effect on fatigue, headache, indisposition and dyspnoea; Evaluate the amount of adverse events and side effects; Evaluate effect on blood lipids and weight; Evaluate the patient's perceived benefit from treatment; Evaluate medical staff's perceived benefit from treatment; Evaluate adherence/adherence to Atacand. Examinations: Evaluation according to standard practice.
Sampling: Sampling according to standard practice.
Treatment information: Transfer of information related to study treatment to and from the patient: effect, adherence, symptom, adverse events, knowledge about treatment.

Communication tool: The invention is implemented in the framework of the communication tool CQ (CircadianQ, Stockholm, Sweden). CQ (Circadian Questions) is an interactive mobile follow-up service that can be used i.e. in follow-up or support in medical treatment. CQ runs on the respondent's own mobile phone and the set of questions are presented by the mobile phone at predefined points in time. Structured answers can be given through VAS (Visual Analogue Scale), which are easy to answer and gives illustrative graphs. Questions relating to multiple choices, integers and decimal numbers may also be posed, and encouragement, recommendations and information may be provided.

The goal of the study is to substantiate that the use of the invention can:
- Increase therapeutic clinical effects of Atacand
- Decrease the amount of adverse events and side effects for the patients during the study period
- Improve target compliance for risk factors for Hypertension
- Improve adherence/compliance to Atacand

The study project is an open randomised study. The project comprises a total of 400 patients that will be offered access to CQ with a specific question-feedback model. A control group is randomly chosen to be followed and receive only standard treatment and support from nurses. The patients will continuously during 6 months receive questions, support and information through their mobile phones in accordance with the set of questions specified below. The answers are registered in a database from where the set of functions will operate in order to create feedback.

The feedback will consist of the following components:
- Graphs based upon the answers from the patient. Visualizing in a structured manner correlations between different questions, e.g. the results on health status or actual level of blood pressure when the patient is adherent to the prescribed regimen of Atacand. In the graphs the answers from the patient will, for instance, be visualized over time.
- The graphs will be illustrated using trend lines. These will appear when the patient has been answering questions for more than a month.
- The patient's evolvement over time is illustrated, for instance in graphs, in relation to realistic health targets. Health targets for the levels of blood pressure over time will be developed. The targets could be individualized for each patient, depending on the amount of available information and circumstances for the patients.
- The patient will be sent short messages based upon his/her health evolvement. These messages will be sent as encouraging and motivating information when the patient:
   o Has fulfilled something positive, such as been adherent to Atacand or achieved a stable and positive level of the measured blood pressure.
   o Needs positive feedback in order to improve their behaviour, for example to become more adherent to Atacand or other important issues, such as the level of performed physical activity.
- In relation to each patient's evolvement and the given answers, future predictions concerning his/her health status will be developed. The future predictions will visualize possible scenarios for the patient based upon how the patient will evolve in some critical issues, for example the compliance/adherence to Atacabd in relation to measured level of blood pressure.
- The set of functions concerning possible adverse events and side effects for Atacand will be implemented. The possible adverse events and/or side effects are visualized for the patient depending on the safety concern. Possible adverse events will be sent by messages and possible side effects will be visualized in context with the given answers, for example in different graphs or other illustrations. The possible adverse events will be sent to health responsible as well.
- The feedback will be possible to individualize, i.e. to remove information if that seems motivated.

The patients will be recommended to adjust their dose of Atacand if necessary.

Patients at the clinical practice with hypertension are informed about the study and the possibility to enter the study. Patients willing to participate are consecutively included. Patients will receive a short introduction of the communication tool. If necessary, patients will be able to contact technical support for the tool. If a patient included by medical staff does not start using the tool, the tool will automatically contact the patient to offer technical support, subject to approval of the patient.

The patients then answer the questions according to a predefined schedule during the course of the study or until the patient chooses to abort participation. The questionnaire regimen is possible to individualize, which is, for example, to remove some questions and to change the specific time when they will show up on the patient's mobile phone. Patients are informed that they shall contact medical staff if they become acutely ill or if their condition seriously deteriorates.

At the beginning the patient fills out a questionnaire relating to basic facts about his or her personal situation and health situation, facts about the primary and secondary aims of the study, the blood pressure is measured, consent to staff to retrieve data from the patient's medical records and to participate in the study, etc. After 6 months the same questions are asked again and the intra-individual change is calculated.

Criteria for inclusion:
- Hypertension
- Access to a mobile telephone
- Ability to follow the CQ programme

Criteria for exclusion:
- Patients who cannot read and/or understand the principal language of the study
- Patients with severely reduced cognitive abilities
- Patients with reduced vision leading to an inability to answer the questions

### Starting set of questions and question schedule, Atacand

Questions, twice a week during the study period
1. How do you feel?
   (VAS; 0=Extremely bad, 10=Extremely good)
2. What is your Systolic blood pressure, measured at home today?
   (numeric)
3. What is your Diastolic blood pressure, measured at home today?
   (numeric)
4. Have you been exercising today?
   (VAS; 0=Not at all, 10=Extremely much)
5. Do you take Atacand according to the prescription?
   (yes / partially / no)

Questions once per week during the study period
1. Have you, during the last week, felt something of the following?
   (dizziness / headache / fatigue / pounding heartbeats / swelling ankles / chest pain / fainting)
2. Your weight today?
   (numeric)
3. Your quality of life as regards to health?
   (VAS; 0=Extremely bad QoL, 10=Extremely good QoL)
4. Are you experiencing stress right now?
   (VAS; 0=Not at all, 10=Extremely much)
5. Do you have any of the following severe allergic reactions to Atacand?
   (rash / hives / itching / difficulty breathing / tightness in the chest / swelling of the mouth, face, lips, or tongue / hoarseness)
6. Do you have any of the following Atacand severe side effects?
   (change in the amount of urine produced / chest pain / dark urine / difficulty swallowing / fast, slow, or irregular heartbeat / fever, chills, or persistent sore throat / muscle pain or cramps / severe or persistent stomach pain / symptoms of low blood pressure / unusual bruising or bleeding / yellowing of the eyes or skin)

Questions every second week during the study period
1. Please keep using Atacand even if you feel fine. High blood pressure often has no symptoms
2. Do you have any of the following most common side effects for Atacand?
   (Back pain / dizziness / upper respiratory tract infection)

Questions once per month during the study period
1. Did you feel well rested this morning?
   (VAS; 0=Not at all rested, 10=Extremely well rested)
2. Are you satisfied with your treatment?
   (VAS; 0=Not at all satisfied, 10=Extremely satisfied)
3. Please avoid drinking alcohol while using Atacand. How many of standard measures of alcohol have you been drinking the last week?
   (numeric)
4. Portion size of breakfast today?
   (VAS; 0=Extremely small, 10=Extremely large)
5. Cooked meal for lunch today?
   (yes/no)
6. Portion size of lunch today?
   (VAS; 0=Extremely small, 10=Extremely large)
7. Portion size of dinner today?
   (VAS; 0=Extremely small, 10=Extremely large)
8. Atacand might interact with other medications. Do you take any of the following medication, as well?
   (Diuretics (eg, furosemide, hydrochlorothiazide) / ACE inhibitors (eg, lisinopril) / potassium-sparing diuretics (eg, spironolactone, triamterene) / NSAID:s (eg, celecoxib, ibuprofen, indomethacin) / Lithium)
9. Remember to stop using Atacand and tell your doctor right away if you become pregnant during treatment

Spontaneous questions, which could be answered by the patient whenever he/she wish:
1. How do you feel?
   (VAS; 0=Extremely bad, 10=Extremely good)
2. Have you, during the last week, felt something of the following?
   (swelling of the hands, face, lips, eyes throat, or tongue / difficulty swallowing or breathing / hoarseness / pounding heartbeats / chest pain)

## Claims

1. A kit of parts comprising
- a pharmaceutical product; and
- a computer program product comprising instructions causing a computer to perform a method comprising the steps
o providing a patient with a set of questions according to a question schedule, wherein said set of questions is specific to the pharmaceutical product;
o collecting answers to said questions from said patient;
o subjecting said answers to a set of functions specific for the set of questions and the pharmaceutical product thereby generating patient-specific feedback information; and
o providing said feedback information to the patient.

2. A kit according to claim 1, wherein the clinical relevance of the combination of said set of questions and said set of functions has been validated in clinical trials.

3. A kit according to claim 1, wherein said set of questions and said set of functions are related to patient compliance to a preferred or prescribed dosage and/or administration regimen of said pharmaceutical product.

4. A kit according to claim 1, wherein said set of questions and said set of functions are related to an indication of possible occurrence or development of an adverse event and/or side effect.

5. A kit according to claim 1, wherein said set of questions and said set of functions are related to the patient's quality of life.

6. A kit according to claim 1, wherein at least a subset of the set of questions is related to the actual administration; actual dosage; perceived and/or measured therapeutic effects; test results and/or perceived quality of life.

7. A kit according to claim 1, wherein the method further comprises subjecting said answers to a set of functions specific for the set of questions and the pharmaceutical product thereby generating an updated question schedule, wherein said set of functions optionally use Computer Adaptive Testing and/or Item Response Theory.

8. A kit according to claim 1, wherein the computer program product comprises instructions causing a computer to perform a method comprising the steps
o providing at least one further respondent in addition to said patient with a second set of questions according to a second question schedule, wherein said second set of questions is specific to the pharmaceutical product;
o collecting answers to said questions from said further respondent;
o subjecting said answers to a second set of functions specific for the second set of questions and the pharmaceutical product thereby generating patient-specific feedback information;
o providing said feedback information to the patient and, optionally, to the further respondent.

9. A kit according to claim 1,wherein said set of functions include functions selected from the group consisting of: calculations of target parameters and trend lines; prediction of development of a condition; rules and thresholds for defining when to give notifications.

10. A kit according to claim 1, wherein said computer program product comprises instructions causing a computer to provide feedback selected from graphs, diagrams, graphical illustrations and text messages.

11. A kit according to claim 1, wherein said method provides feedback only to the patient, or to the patient and to other individuals.

12. A kit according to claim 1, wherein said computer program product is provided on a physical medium or by means or instructions for accessing and installing the computer program product on a computer.

13. A kit according to claim 13, wherein said computer program product is provided by means or instructions for accessing and installing the computer program product on a computer and said kit further comprises an identifier unique to the kit.

14. The computer program product of claim 1.

15. The method of claim 1.
